# EUROPEAN PATENT APPLICATION

(11) **EP 4 748 356 A1**
(43) Date of publication of application: **27.05.2026**
(21) Application number: 24849132.6
(22) Date of filing: 29.07.2024
(51) Int. Cl.: A61F 13/15

(54) **METHOD FOR MANUFACTURING ABSORBENT ARTICLE AND SYSTEM FOR MANUFACTURING ABSORBENT ARTICLE**

(30) Priority: 01.08.2023 JP 2023125826
(71) Applicant: Zuiko Corporation, Ibaraki-shi Osaka 5670082 (JP)
(72) Inventor: UMEBAYASHI, Toyoshi, Ibaraki-shi, Osaka 567-0082 (JP); HONGE, Susumu, Ibaraki-shi, Osaka 567-0082 (JP)
(74) Representative: SSM Sandmair
(86) International application number: PCT/JP2024/026955
(87) International publication number: WO 2025/028481

(57) **Abstract**

To provide a method for manufacturing an absorbent article that can reduce installation costs and floor space required for installing apparatuses. The method for manufacturing an absorbent article includes: a continuous body forming step; a setting step; a continuous body transporting step; a first manufacturing step; and a second manufacturing step. In the continuous body forming step, a continuous body having a plurality of absorbent bodies in a state where the absorbent bodies are connected in series is formed. In the setting step, a transport path of the continuous body is set to either a first transport path or a second transport path. In the continuous body transporting step, the continuous body is transported along the set transport path. In the first manufacturing step, the continuous body transported along the first transport path is cut and separated into individual absorbent bodies and a first absorbent article including the separated individual absorbent body is manufactured. In the second manufacturing step, the continuous body transported along the second transport path is cut and separated into individual absorbent bodies and, using the separated individual absorbent body, a second absorbent article of a different type from the first absorbent article is manufactured.

## Description

### Technical Field

The present invention relates to a method for manufacturing an absorbent article.

### Background Art

As described in PTL 1 (WO 2019/150802) and PTL 2 (Japanese Patent Application Publication No. 2017-64131), methods of manufacturing an absorbent article using an absorbent body are known.

Different types of such absorbent articles may be manufactured in a same factory.

### Citation List

### Patent Literature

PTL 1: WO 2019/150802
PTL 2: Japanese Patent Application Publication No. 2017-64131

### Summary of Invention

### Technical Problem

When manufacturing different types of absorbent articles such as a tape-type diaper described in PTL 1 and a pants-type diaper described in PTL 2, providing separate apparatuses for manufacturing each absorbent article increases costs and floor space required for installing manufacturing apparatuses.

### Solution to Problem

A method for manufacturing an absorbent article according to one aspect includes: a continuous body forming step; a setting step; a continuous body transporting step; a first manufacturing step; and a second manufacturing step. In the continuous body forming step, a continuous body having a plurality of absorbent bodies in a state where the absorbent bodies are connected in series is formed. In the setting step, a transport path of the continuous body is set to either a first transport path or a second transport path. In the continuous body transporting step, the continuous body is transported along the set transport path. In the first manufacturing step, the continuous body transported along the first transport path is cut and separated into individual absorbent bodies and a first absorbent article including the separated individual absorbent body is manufactured. In the second manufacturing step, the continuous body transported along the second transport path is cut and separated into individual absorbent bodies and, using the separated individual absorbent body, a second absorbent article of a different type from the first absorbent article is manufactured.

A method for manufacturing an absorbent article according to another aspect includes: a continuous body forming step; a separating step; a first manufacturing step; a second manufacturing step; and a switching step. In the continuous body forming step, a continuous body having a plurality of absorbent bodies in a state where the absorbent bodies are connected in series is formed. In the separating step, the continuous body is cut and separated into individual absorbent bodies. In the first manufacturing step, using the absorbent bodies, a first absorbent article is manufactured. In the second manufacturing step, using the absorbent bodies, a second absorbent article that differs from the first absorbent article is manufactured. In the switching step, a supply destination of the absorbent bodies separated in the separating step is switched between the first manufacturing step and the second manufacturing step.

### Advantageous Effects of Invention

The method for manufacturing an absorbent article according to the present invention enables reducing costs and floor space required for installing apparatuses.

### Brief Description of Drawings

[Fig. 1] Fig. 1 is a schematic view of a system for manufacturing an absorbent article used in a method for manufacturing an absorbent article according to one embodiment of the invention of the present application.
[Fig. 2] Fig. 2 is a diagram schematically showing the system for manufacturing an absorbent article of Fig. 1 with a part of the apparatuses being omitted.
[Fig. 3] Fig. 3 is a schematic flow chart showing the method for manufacturing an absorbent article according to the one embodiment of the invention of the present application.
[Fig. 4] Fig. 4 is a schematic view explaining manufacturing steps of a tape-type disposable diaper.
[Fig. 5] Fig. 5 is a schematic view explaining manufacturing steps of a pants-type disposable diaper.
[Fig. 6] Fig. 6 is a schematic flow chart showing a method for manufacturing an absorbent article according to another embodiment of the invention of the present application.
[Fig. 7] Fig. 7 is a schematic view of a system for manufacturing an absorbent article used in the method for manufacturing an absorbent article according to the other embodiment of the invention of the present application.
[Fig. 8] Fig. 8 is a diagram schematically showing the system for manufacturing an absorbent article of Fig. 6 with a part of the apparatuses being omitted.
[Fig. 9] Fig. 9 is a schematic view explaining manufacturing steps of a pants-type disposable diaper according to a modification.

### Description of Embodiments

Hereinafter, embodiments of a method for manufacturing an absorbent article will be described with reference to the drawings. A first direction referred to in the following description is a longitudinal direction of each absorbent body.

Note that the embodiments described below are merely examples of the invention of the present application and are not intended to limit the scope of the invention of the present application. Those skilled in the art will understand that various modifications to the following embodiments are possible without departing from the spirit and scope of the invention of the present application as described in the appended claims.

### [First embodiment]

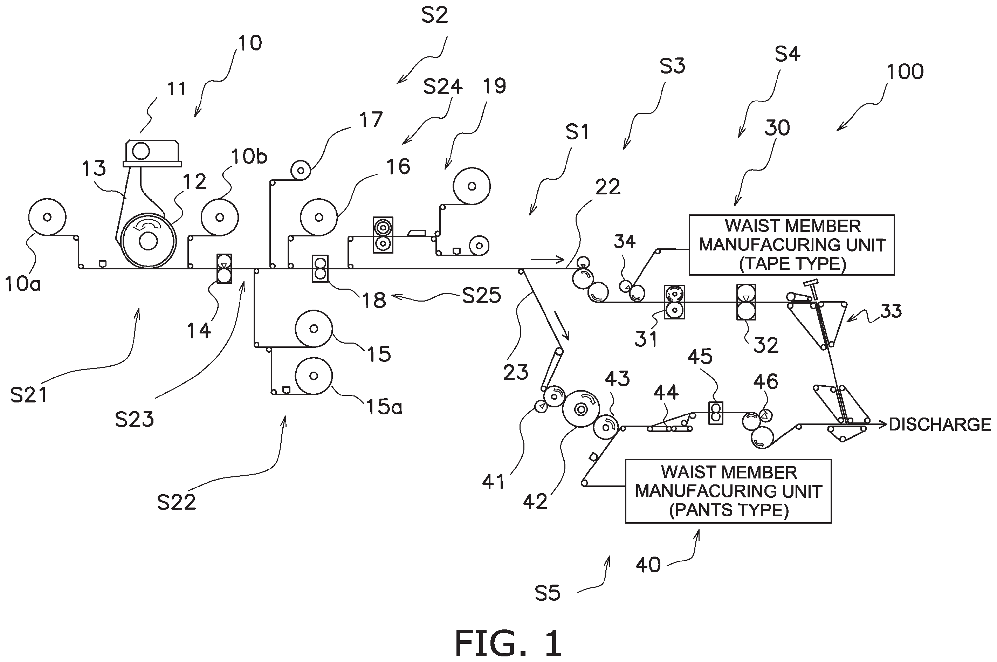

A method for manufacturing an absorbent article according to a first embodiment will be described with reference to Figs. 1 to 5.

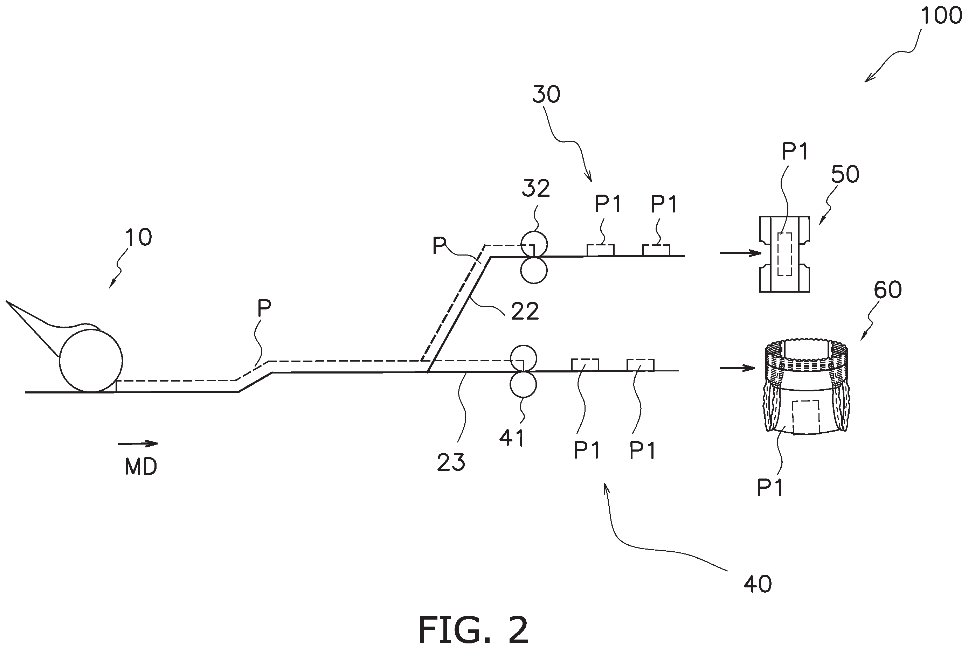

As shown in Figs. 1 and 2, the method for manufacturing an absorbent article according to the first embodiment of the invention of the present application uses a system 100 for manufacturing an absorbent article. The system 100 for manufacturing an absorbent article includes an absorbent core manufacturing apparatus 10, a first manufacturing apparatus 30, and a second manufacturing apparatus 40. The first manufacturing apparatus 30 manufactures a first absorbent article 50 using an absorbent body P1 to be described later, and the second manufacturing apparatus 40 manufactures, using the absorbent body P1, a second absorbent article 60 of a different type from the first absorbent article 50.

The absorbent body P1 is an article used, for example, in disposable diapers, urine-absorbing pads, and the like and designed to absorb bodily fluids such as urine. In the present embodiment, the first absorbent article 50 is a tape-type diaper and the second absorbent article 60 is a pants-type diaper. Note that a tape-type diaper is a diaper in which an absorbent body is arranged under the crotch when being worn and a front waist-side attachment member and a rear waist-side attachment member are fastened with tape.

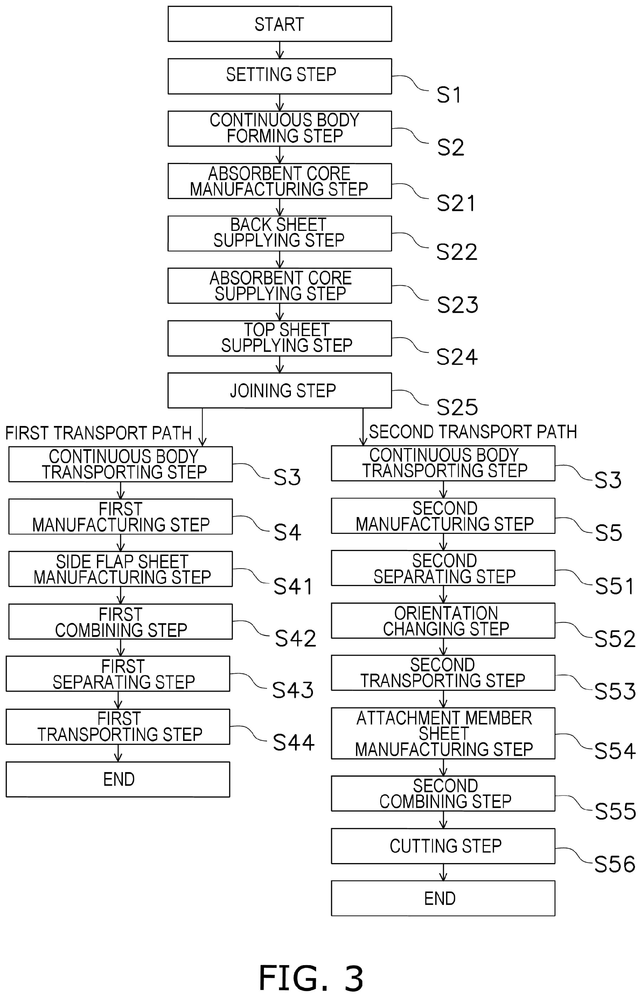

As shown in Fig. 3, the method for manufacturing an absorbent article includes: a setting step S1; a continuous body forming step S2; a continuous body transporting step S3; a first manufacturing step S4; and a second manufacturing step S5. Each step will be described below.

### (1) Setting step S1

In the setting step S1, a transport path of a continuous body P of the absorbent body P1 to be described later is set to either a first transport path 22 or a second transport path 23. While a method for setting a transport path is not particularly limited, for example, before start of production of the first absorbent article 50 or the second absorbent article 60, an operator manually winds the continuous body P of the absorbent body P1 around a guide roll or the like so that the continuous body P is fed to the first manufacturing apparatus 30 or the second manufacturing apparatus 40 and transported in the first manufacturing apparatus 30 or the second manufacturing apparatus 40.

In particular, when setting the transport path of the continuous body P of the absorbent body P1 to the first transport path 22, the operator winds the continuous body P of the absorbent body P1 around a guide roll or the like (not illustrated) that guides the continuous body P so as to pass through an end-side sealing machine 31, a product cutter 32, and a double-folding apparatus 33.

When setting the transport path of the continuous body P of the absorbent body P1 to the second transport path 23, the operator winds the continuous body P of the absorbent body P1 around a guide roll or the like (not illustrated) that guides the continuous body P so as to pass through an inner cutter 41, an orientation changing apparatus 42, a joining apparatus 43, a double-folding apparatus 44, a side sealing apparatus 45, and a product cutter 46.

### (2) Continuous body forming step S2

In the continuous body forming step S2, the continuous body P having a plurality of absorbent bodies P1 in a state where the absorbent bodies P1 are connected in series is formed. The continuous body P of the absorbent bodies P1 is an article obtained by forming pulp fibers into a predetermined shape.

The forming step S2 of the continuous body P mainly includes an absorbent core manufacturing step S21, a back sheet supplying step S22, an absorbent core supplying step S23, a top sheet supplying step S24, and a joining step S25.

The absorbent core manufacturing step S21 includes a lower cover supplying step, a laminating step, an upper cover supplying step, and a cutting step.

In the lower cover supplying step, a continuous sheet to become the lower cover is transported and supplied along a longitudinal direction thereof. The supplied continuous sheet to become the lower cover is transported in a state of being subjected to predetermined tension in the longitudinal direction thereof until the continuous sheet is cut in the cutting step. The continuous sheet to become the lower cover is supplied from a lower cover supplying apparatus 10a.

In the laminating step, the absorbent core manufacturing apparatus 10 laminates water-absorbing pulp fibers with respect to the continuous sheet to become the lower cover.

The absorbent core manufacturing apparatus 10 mainly includes a pulverizing apparatus 11, a fiber laminating apparatus 12, and a duct 13. The pulverizing apparatus 11 pulverizes a pulp sheet formed by agglutinating pulp fibers into a sheet form to generate pulp fibers. In other words, the pulverizing apparatus 11 is an apparatus that separates (defibrates) pulp fibers formed in a sheet form into flocs. The duct 13 functions as a pulp fiber passageway that guides the pulp fibers generated by the pulverizing apparatus 11 from the pulverizing apparatus 11 to the fiber laminating apparatus 12. The fiber laminating apparatus 12 laminates the pulp fibers pulverized by the pulverizing apparatus 11 on the lower cover.

In the upper cover supplying step, the pulp fibers laminated on the lower cover are covered by a continuous sheet to become an upper cover. The continuous sheet to become the upper cover is supplied from an upper cover supply 10b.

In the cutting step, a laminate enclosed by the upper cover and the lower cover is cut using a mat cutter 14 to manufacture individually independent absorbent cores.

In the back sheet supplying step S22, a first continuous web to form a laminated back sheet and a second continuous web to form a waterproof sheet are supplied continuously. Here, the first continuous web and the second continuous web will be referred to as a back sheet web group. Note that in the back sheet web group, the second continuous web to form the waterproof sheet is laminated on a surface facing a skin side of the first continuous web to form the back sheet. The back sheet is constituted of a liquid-impermeable resin sheet that covers a non-skin surface of the absorbent core. In the present invention, the "non-skin surface" refers to an outside surface of the diaper that is opposite to a skin surface, that does not come into contact with the wearer's skin during use, and that does not face the wearer's skin. In addition, in the present invention, the "skin surface" refers to an inside surface of the diaper that directly or indirectly comes into contact with the wearer's skin during use and that directly or indirectly faces the wearer's skin. The first continuous web is supplied from a back sheet supplying apparatus 15. The second continuous web is supplied from a waterproof sheet supplying apparatus 15a.

In the absorbent core supplying step S23, the transported individual absorbent cores are supplied onto the back sheet web group. In the absorbent core supplying step S23, the absorbent cores are supplied to the back sheet web group at predetermined intervals in a transport direction of the back sheet web group.

In the top sheet supplying step S24, a third continuous web to form the top sheet is continuously supplied above the absorbent core arranged on the back sheet web group. In summary, in the top sheet supplying step S24, the third continuous web is supplied to the laminate of the back sheet web group constituted of the first continuous web and the second continuous web and the absorbent cores so that the absorbent cores are arranged between the back sheet web group and the third continuous web. The third continuous web is supplied from a top sheet supplying apparatus 16. The top sheet is constituted of a liquid-permeable nonwoven fabric.

In the joining step S25, the third continuous web to form the top sheet and the back sheet web group constituted of the first continuous web and the second continuous web are joined to each other by a joining apparatus 18 in a laminated state where the absorbent cores are sandwiched between the third continuous web and the back sheet web group to manufacture the continuous body P of the absorbent body P1.

The joining step S25 will be described in greater detail.

In the joining step S25, when joining the back sheet web group and the third continuous web to each other, an elastic member is supplied from an elastic member supplying apparatus 17 to end portions of the third continuous web and the back sheet web group in a transverse direction. The elastic member supplying apparatus 17 supplies the elastic member to the end portions of the third continuous web and the back sheet web group in the transverse direction in a state where the elastic member is stretched in the longitudinal direction. The elastic member is a member for causing the absorbent body P1 to adhere closely to the wearer's crotch portion when the wearer puts on the absorbent articles 50 and 60. For example, an elastic cord as well as materials in thread or strip form containing polyurethane foam can be adopted as the elastic member. Although joining methods are not limited thereto, the joining apparatus 18 intermittently thermally fuses the back sheet web group and the third continuous web to each other using the joining apparatus 18 in the longitudinal direction of the back sheet web group and the third continuous web.

Furthermore, in the joining step S25, cuffs formed using a cuff supplying apparatus 19 are arranged at both end portions of the top sheet in the transverse direction. The cuffs function as three-dimensional gathers that prevent bodily fluids of the wearer from leaking out of the crotch portion.

Note that while the first absorbent article 50 and the second absorbent article 60 are diapers in the present embodiment, when the first absorbent article 50 and the second absorbent article 60 are not diapers (for example, when they are urine-absorbing pads or the like), apparatuses related to supplying an elastic member and forming cuffs such as the elastic member supplying apparatus 17 and the cuff supplying apparatus 19 in the joining step S25 need not be provided. When only one of the first absorbent article 50 and the second absorbent article 60 is a diaper and the other is not, although apparatuses related to supplying an elastic member and forming cuffs are provided, the apparatuses need not be used.

### (3) Continuous body transporting step S3

In the continuous body transporting step S3, the continuous body P is transported along the set transport path. The first transport path 22 and the second transport path 23 are arranged in the continuous body transporting step S3.

The first manufacturing apparatus 30 manufactures the first absorbent article 50 using the continuous body P transported along the first transport path 22. When the first transport path 22 is set in the setting step S1, the continuous body P of the absorbent body P1 is supplied onto the first transport path 22 and transported to the first manufacturing apparatus 30. At this point, the continuous body P of the absorbent body P1 is not supplied onto the second transport path 23.

The second manufacturing apparatus 40 manufactures the second absorbent article 60 using the continuous body P transported along the second transport path 23. When the second transport path 23 is set in the setting step S1, the continuous body P of the absorbent body P1 is supplied onto the second transport path 23 and transported to the second manufacturing apparatus 40. At this point, the continuous body P of the absorbent body P1 is not supplied onto the first transport path 22.

Note that when switching a transport destination of the continuous body P of the absorbent body P1 (in other words, when the worn article to be manufactured is switched between the first absorbent article 50 and the second absorbent article 60), the step (setting step S1) of newly winding the continuous body P of the absorbent body P1 around the first manufacturing apparatus 30 or the second manufacturing apparatus 40 is performed once again.

### (4) First manufacturing step S4

In the first manufacturing step S4, the continuous body P transported along the first transport path 22 is cut and separated into individual absorbent bodies P1 and the first absorbent article 50 including the separated individual absorbent body P1 is manufactured.

In the first manufacturing step S4, the first absorbent article 50 is manufactured using the first manufacturing apparatus 30. Although arrangements are not limited thereto, for example, the first manufacturing apparatus 30 is arranged above the second manufacturing apparatus 40.

The first manufacturing apparatus 30 manufactures the first absorbent article 50 using the continuous body P of the absorbent body P1 transported along the first transport path 22. In the present embodiment, the first absorbent article 50 is a tape-type diaper as described earlier.

Hereinafter, the first manufacturing step S4 in a case where the first absorbent article 50 is a tape-type diaper will be described.

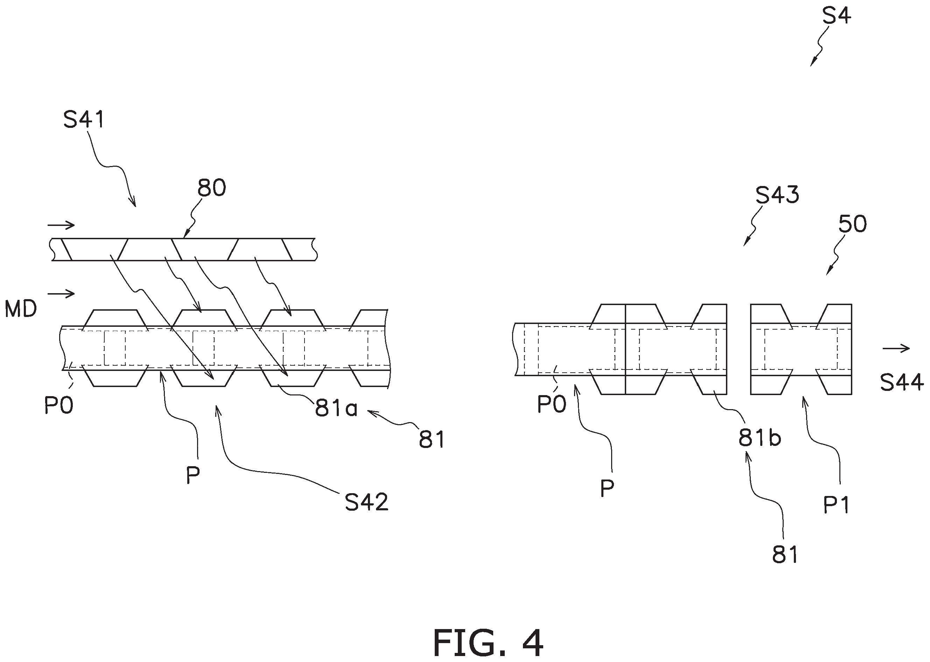

As shown in Fig. 4, the first manufacturing step S4 mainly includes a side flap sheet manufacturing step S41, a first combining step S42, a first separating step S43, and a first transporting step S44.

In the side flap sheet manufacturing step S41, a side flap sheet to become a first attachment member 81 to be attached around a person's waist after undergoing a subsequent step is manufactured.

The side flap sheet manufacturing step S41 will be described in detail.

In the side flap sheet manufacturing step S41, an outer flap cutter 34 (refer to Fig. 1) cuts out a trapezoidal side flap sheet 81a (refer to Fig. 4) by cutting a strip-shaped sheet 80 in a direction that intersects both a transport direction and a direction orthogonal to the transport direction. In the side flap sheet manufacturing step S41, trapezoids with a longer side arranged on a right side and trapezoids with a longer side arranged on a left side when viewed along the transport direction are alternately formed.

In the first combining step S42, the side flap sheet 81a and the continuous body P of the absorbent body P1 are combined. Specifically, long side portions of a trapezoidal first attachment member 81 are joined to edge portions on both sides of the continuous body P of the absorbent body P1 in the transverse direction using the end-side sealing machine 31. In the first combining step S42, the trapezoid formed in the side flap sheet manufacturing step S41 of which the long side is arranged on the right side in the transport direction of the sheet 80 is joined to the left side of the transported continuous body P of the absorbent body P1, and the trapezoid formed in the side flap sheet manufacturing step S41 of which the long side is arranged on the left side in the transport direction of the sheet 80 is joined to the right side of the transported continuous body P of the absorbent body P1.

Note that in the first combining step S42, side flap sheets 81a adjacent to each other in a transport direction MD is arranged separated by a predetermined distance in the transport direction MD.

In the first separating step S43, the product cutter 32 cuts the continuous body P, which is transported along the first transport path 22 and to which the side flap sheet 81a has been joined, to separate the continuous body P into individual absorbent bodies P1 of which a longitudinal direction is the first direction that coincides with the transport direction MD. When cutting the continuous body P, the product cutter 32 cuts a central portion of the side flap sheet 81a joined to the continuous body P in the transport direction MD. Accordingly, each side flap sheet 81a is separated into two pieces and each piece is arranged on an adjacent absorbent body P1 as a side flap 81b, respectively. As shown in Fig. 4, four side flaps 81b provided in each absorbent body P1 constitute the first attachment member 81 to be attached around a person's waist. The four side flaps 81b that function as the first attachment member 81 are positioned on the wearer's abdomen and back when being worn by the wearer. In addition, when being worn by the wearer, a pair of side flaps 81b lined up in the longitudinal direction of the first attachment member 81 are arranged to overlap each other so that the diaper as the first absorbent article 50 covers the entire waist. The side flaps 81b that overlap each other are fixed to each other by a tape (hook-and-loop fastener) (not illustrated) attached to at least a part of the side flaps 81b so that the first absorbent article 50 is fixed to the wearer.

In the first transporting step S44, the individual absorbent bodies P1 after separation (in other words, the first absorbent article 50 in an unfolded state) are transported such that the first direction being the longitudinal direction of the absorbent bodies P1 coincides with the transport direction of the absorbent bodies P1. The transported absorbent bodies P1 are folded in half along center lines of the absorbent bodies P1 in the longitudinal direction using the double-folding apparatus 33.

As described above, the first manufacturing apparatus 30 forms a tape-type disposable diaper that is the first absorbent article 50.

### (5) Second manufacturing step S5

As shown in Figs. 1 and 2, in the second manufacturing step S5, the continuous body P transported along the second transport path 23 is cut and separated into individual absorbent bodies P1 and the second absorbent article 60 is manufactured using the separated individual absorbent body P1. The second absorbent article 60 is an absorbent article of a different type from the first absorbent article 50. In the present embodiment, the second absorbent article 60 is a pants-type diaper as described earlier.

Hereinafter, the second manufacturing step S5 in a case where the second absorbent article 60 is a pants-type diaper will be described.

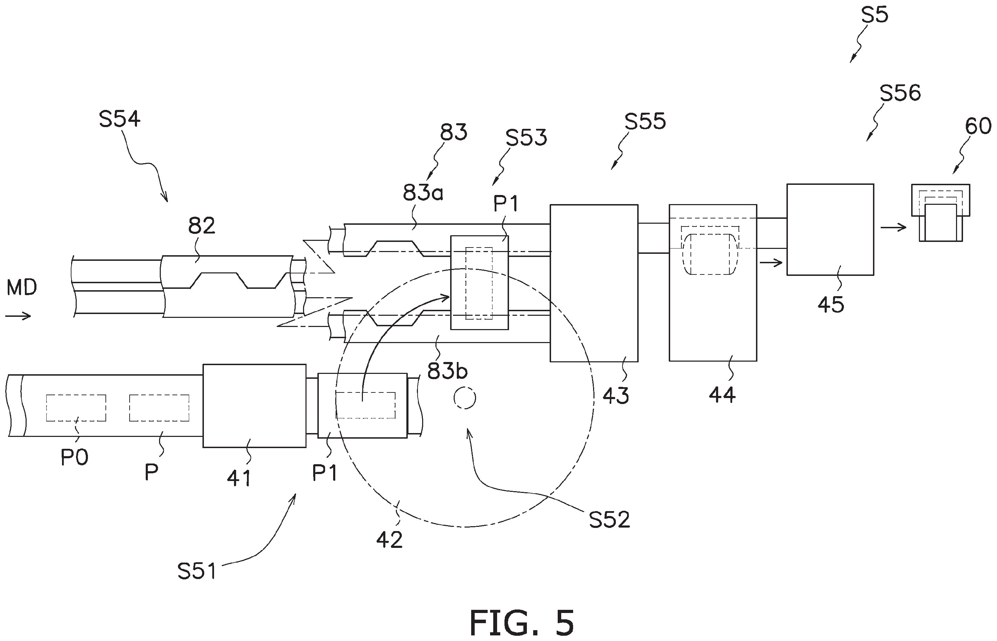

As shown in Fig. 5, the second manufacturing step S5 includes a second separating step S51, an orientation changing step S52, a second transporting step S53, an attachment member sheet manufacturing step S54, a second combining step S55, and a cutting step S56.

As shown in Figs. 1 and 2, the second manufacturing apparatus 40 is used in the second manufacturing step S5. In the second separating step S51, the inner cutter 41 of the second manufacturing apparatus 40 cuts the continuous body P transported along the second transport path 23 and separates the continuous body P into absorbent bodies P1. Accordingly, the continuous body P of the absorbent body P1 becomes mutually independent absorbent bodies P1.

In the orientation changing step S52, after separation in the second separating step S51, the orientation changing apparatus 42 rotates, by 90°, an orientation of each absorbent body P1 being transported so that the first direction being the longitudinal direction of the absorbent bodies P1 and the transport direction of the absorbent bodies P1 coincide with each other. In other words, in the orientation changing step S52, the first direction being the longitudinal direction of the absorbent bodies P1 is made orthogonal to the transport direction of the absorbent bodies P1.

In the second transporting step S53, the individual absorbent bodies P1 after separation are transported such that the first direction and the transport direction MD of the absorbent bodies P1 are orthogonal to each other.

In the attachment member sheet manufacturing step S54, as shown in Fig. 5, a first sheet 83a and a second sheet 83b to become a second attachment member 83 to be attached around a person's waist after undergoing a subsequent step is manufactured. Note that the first sheet 83a becomes a member to cover a front waist of the wearer of the second attachment member 83 after undergoing a subsequent step and the second sheet 83b becomes a member to cover a rear waist of the wearer of the second attachment member 83 after undergoing a subsequent step.

The first sheet 83a and the second sheet 83b are cut out from a strip-shaped sheet 82. Specifically, the strip-shaped sheet 82 is cut into the first sheet 83a and the second sheet 83b along the longitudinal direction by a cutter (not illustrated) arranged approximately at the center in a width direction. The formed first sheet 83a and second sheet 83b are fed along the longitudinal direction while being subjected to tension of a predetermined magnitude.

The first sheet 83a and the second sheet 83b cut from the sheet 82 are widened outward in the width direction from a center line that extends along the longitudinal direction of the sheet 82 at the center of the sheet 82 in the width direction, and are then fed parallel to each other along the longitudinal direction of each sheet.

Note that in the present embodiment, the strip-shaped sheet 82 is cut by the cutter so as to form a wave pattern along the longitudinal direction as shown in Fig. 5. As a result, when viewed from a direction perpendicular to a surface of the sheet 82 (from the viewpoint in Fig. 5), protrusions that project toward an inner side of the sheet 82 and depressions that are recessed toward an outer side of the sheet 82 are alternately formed on cut sides of the first sheet 83a and the second sheet 83b. Note that during transportation of the first sheet 83a and the second sheet 83b, position alignment is performed so that the first sheet 83a and the second sheet 83b are line-symmetrical with respect to the center line of the sheet 82. In other words, during transportation of the first sheet 83a and the second sheet 83b, phase alignment is performed so that a position of the depression of the first sheet 83a and a position of the depression of the second sheet 83b coincide with each other and a position of the protrusion of the first sheet 83a and a position of the protrusion of the second sheet 83b coincide with each other in the transport direction of the first sheet 83a and the second sheet 83b.

In the second combining step S55, the first sheet 83a, the second sheet 83b, and the absorbent body P1 are combined. Specifically, in the second combining step S55, the absorbent body P1 is arranged on the first sheet 83a and the second sheet 83b so that the absorbent body P1 spans between the first sheet 83a and the second sheet 83b transported in parallel and that the longitudinal direction of the absorbent body P1 coincides with a direction orthogonal to the transport direction MD of the first sheet 83a and the second sheet 83b. Specifically, in the second combining step S55, the absorbent body P1 is arranged so as to span between the depression that is recessed toward an outer side of the first sheet 83a and the depression that is recessed toward an outer side of the second sheet 83b transported in parallel. In addition, in the second combining step S55, the absorbent body P1, the first sheet 83a, and the second sheet 83b are joined using the joining apparatus 43. For example, ultrasonic joining is used as a joining method. However, the joining method is not limited to ultrasonic joining and may be selected as appropriate.

Furthermore, in the second combining step S55, the absorbent body P1 is folded in half at a center portion in the width direction (a direction orthogonal to the first direction being the longitudinal direction of the absorbent body P1) by the double-folding apparatus 44 so that respective end edges of the first sheet 83a and the second sheet 83b in the first direction being the longitudinal direction of the absorbent body P1 (in other words, respective end edges of the first sheet 83a and the second sheet 83b in a direction orthogonal to the transport direction of the first sheet 83a and the second sheet 83b) overlap with each other in a plan view.

In addition, in the second combining step S55, in a center portion between two absorbent bodies P1 adjacent to each other in the transport direction, the overlapping sheets 83a and 83b are joined by the side sealing apparatus 45 across their entire length in the direction orthogonal to the transport direction of the first sheet 83a and the second sheet 83b, thereby forming a side seal portion.

In the cutting step S56, the first sheet 83a and the second sheet 83b are cut by the product cutter 46 (refer to Fig. 1) at center in a width direction of the formed side seal portion (transport direction of the first sheet 83a and the second sheet 83b). Accordingly, the first sheet 83a becomes a member to cover a front waist of the wearer of the second attachment member 83 and the second sheet 83b becomes a member to cover a rear waist of the wearer of the second attachment member 83. Note that the member formed from the first sheet 83a to cover the front waist of the wearer of the second attachment member 83 and the member formed from the second sheet 83b to cover the rear waist of the wearer of the second attachment member 83 are connected by the side seal portion and cover the entire waist portion of the wearer as a whole.

As described above, the second manufacturing apparatus 40 forms a pants-type disposable diaper that is the second absorbent article 60.

### [Second embodiment]

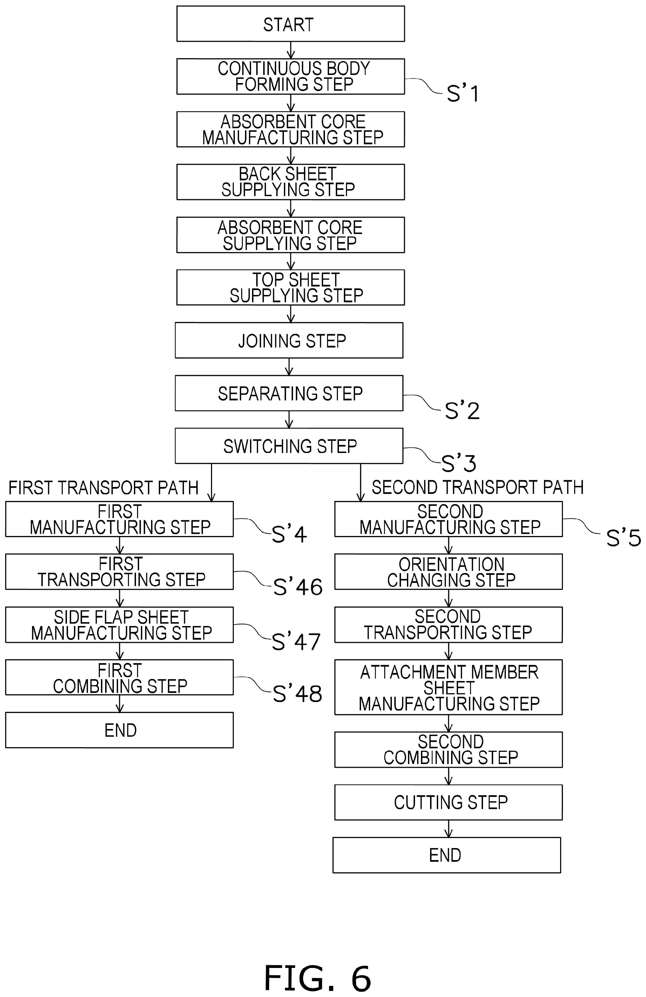

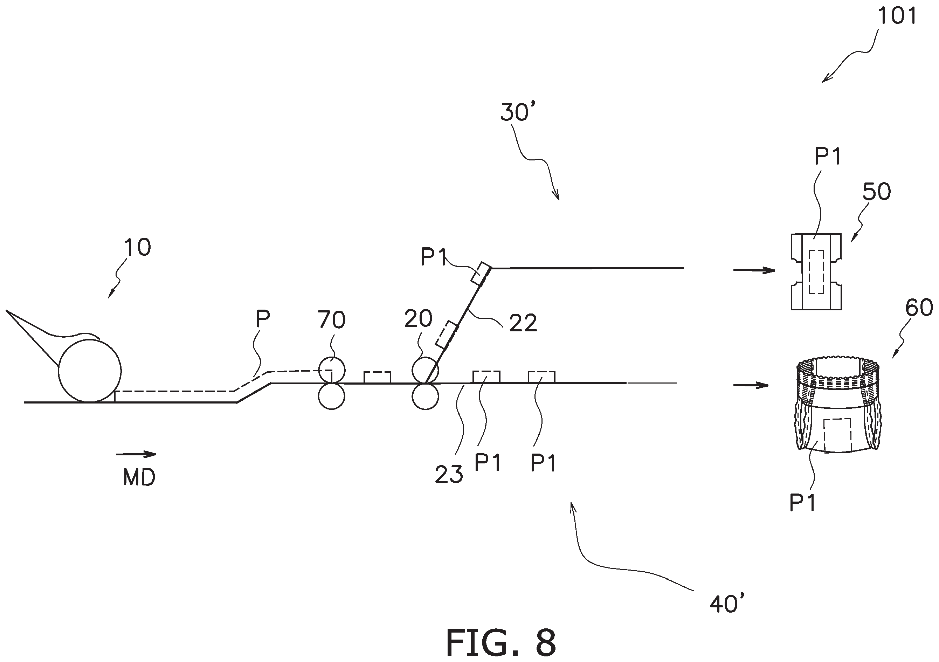

A method for manufacturing an absorbent article according to a second embodiment will be described with reference to Figs. 6 to 8.

First, to outline differences between the manufacturing method according to the first embodiment and a manufacturing method according to the second embodiment, the manufacturing method according to the first embodiment and the manufacturing method according to the second embodiment differ in the following points.

In the manufacturing method according to the first embodiment, the continuous body P of the absorbent body P1 is supplied to the first manufacturing apparatus 30 or the second manufacturing apparatus 40 as it is. In contrast, in the manufacturing method according to the second embodiment, each absorbent body P1 is separated from the manufactured continuous body P and the absorbent body P1 is supplied to the first manufacturing apparatus 30 or the second manufacturing apparatus 40.

As shown in Fig. 6, the method for manufacturing an absorbent article according to the second aspect includes: a continuous body forming step S'1; a separating step S'2; a switching step S'3; a first manufacturing step S'4; and a second manufacturing step S'5. The description of the second embodiment will focus on differences from the first embodiment. Steps and apparatuses of the second embodiment other than those described below are the same as those of the first embodiment.

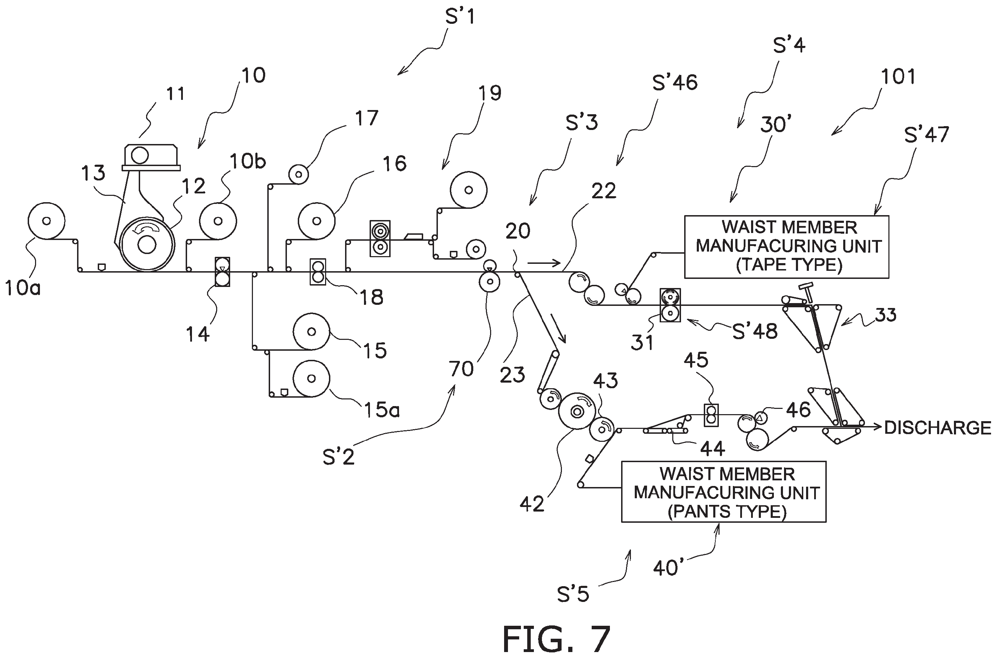

As shown in Fig. 7, the method for manufacturing an absorbent article according to the second embodiment uses a system 101 for manufacturing an absorbent article.

### (1) Continuous body forming step S'1

In the continuous body forming step S'1, the continuous body P having a plurality of absorbent bodies P1 in a state where the absorbent bodies P1 are connected in series is formed. Note that the forming step S'1 of the continuous body P of the absorbent body P1 is similar to the forming step S2 of the continuous body P of the absorbent body P1 according to the first embodiment. A detailed description will be omitted here.

### (2) Separating step S'2

In the separating step S'2, an inner cutter 70 cuts the continuous body P and separates the continuous body P into absorbent bodies P1.

### (3) Switching step S'3

In the switching step S'3, a supply destination of the absorbent bodies P1 separated in the separating step S'2 is switched between the first manufacturing step S'4 and the second manufacturing step S'5. Although switching methods are not limited thereto, for example, a switching apparatus 20 is used to switch absorbent bodies P1.

The switching apparatus 20 is arranged between the absorbent core manufacturing apparatus 10 and the first transport path 22 and the second transport path 23 that are supply destinations of the absorbent bodies P1. The switching apparatus 20 switches the supply destination of the absorbent bodies P1 separated in the separating step S'2 between the first transport path 22 and the second transport path 23 and transports the absorbent bodies P1 to the supply destination.

For example, the switching apparatus 20 changes the supply destination of the absorbent bodies P1 based on an operator command input to a control apparatus (not illustrated) and transports the absorbent bodies P1 to the supply destination. For example, the switching apparatus 20 alternately transports the absorbent bodies P1 to the first transport path 22 and the second transport path 23. Alternatively, for example, the switching apparatus 20 may be configured to continuously transport the absorbent bodies P1 to one of the first transport path 22 and the second transport path 23 until the operator performs a switching operation and change the transport destination of the absorbent bodies P1 to the other of the first transport path 22 and the second transport path 23 once the operator's switching operation is performed.

### (4) First manufacturing step S'4

In the first manufacturing step S'4, the first absorbent article 50 including the absorbent body P1 is manufactured. In the present embodiment as well, the first absorbent article 50 is a tape-type diaper.

In the first manufacturing step S'4, the continuous body P of the absorbent body is manufactured using a first manufacturing apparatus 30'. Unlike the first manufacturing apparatus 30, the first manufacturing apparatus 30' does not receive the continuous body P of the absorbent body P1 and manufacture the first absorbent article 50 but manufactures the first absorbent article 50 using the absorbent body P1 that is transported.

The first manufacturing step S'4 mainly includes a first transporting step S'46, a side flap sheet manufacturing step S'47, and a first combining step S'48.

In the first transporting step S'46, the individual absorbent bodies P1 after separation are transported such that the first direction being the longitudinal direction of the absorbent bodies P1 coincides with the transport direction of the absorbent bodies P1.

In the side flap sheet manufacturing step S'47, the first attachment member 81 to be attached around a person's waist is manufactured. In the side flap sheet manufacturing step S'47, the side flap 81b with a shape similar to that described in the first embodiment is formed. In a similar manner to the first embodiment, each first absorbent article 50 includes four side flaps 81b as the first attachment member 81.

In the first combining step S'48, individually independent side flaps 81c are combined with the absorbent bodies P1 and a tape-type diaper is manufactured as the first absorbent article 50. Specifically, in the first combining step S'48, the side flaps 81c are fused to the edge portions on both sides of the absorbent bodies P1 in the transverse direction and edge portions on both sides of the absorbent bodies P1 in the first direction being the longitudinal direction in a similar manner such as that shown in Fig. 4.

The first absorbent article 50 in an unfolded state is transported such that the first direction being the longitudinal direction of the absorbent bodies P1 coincides with the transport direction of the absorbent bodies P1. The transported absorbent bodies P1 are folded in half along center lines of the absorbent bodies P1 in the longitudinal direction using the double-folding apparatus 33 in a similar manner to the first embodiment.

As described above, the first manufacturing apparatus 30' forms a tape-type disposable diaper that is the first absorbent article 50.

### (5) Second manufacturing step S'5

In the second manufacturing step S'5, the continuous body P of the absorbent body is manufactured using a second manufacturing apparatus 40'. Unlike the second manufacturing apparatus 40, the second manufacturing apparatus 40' manufactures the second absorbent article 60 of a different type from the first absorbent article 50 using the separated individual absorbent body P1.

The second manufacturing step S'5 is similar to the second manufacturing step S5 according to the first embodiment with the exception of not including the second separating step S51. A detailed description will be omitted here.

### <Features>

(1) A method for manufacturing an absorbent article according to a first aspect of the invention of the present application includes: a forming step S2 of forming a continuous body P; a setting step S1; a continuous body transporting step S3; a first manufacturing step S4; and a second manufacturing step S5. In the forming step S2 of forming the continuous body P, the continuous body P having a plurality of absorbent bodies P1 in a state where the absorbent bodies P1 are connected in series is formed. In the setting step S1, a transport path of the continuous body P is set to either the first transport path 22 or the second transport path 23. In the continuous body transporting step S3, the continuous body P is transported along the set transport path. In the first manufacturing step S4, a continuous body P transported along the first transport path 22 is cut and separated into individual absorbent bodies P1 and a tape-type diaper 50 including the separated individual absorbent body P1 is manufactured. In the second manufacturing step S5, a continuous body P transported along the second transport path 23 is cut and separated into individual absorbent bodies P1 and, using the separated individual absorbent body P1, a pants-type diaper 60 of a different type from the tape-type diaper 50 is manufactured. By adopting such a configuration, the method for manufacturing an absorbent article enables reducing costs and floor space required for installing apparatuses.
(2) A method for manufacturing an absorbent article according to a second aspect of the invention of the present application is the method for manufacturing an absorbent article according to the first aspect, in which the first manufacturing step S4 includes a first separating step S43 and a first transporting step S44. In the first separating step S43, the continuous body P transported along the first transport path 22 is cut to separate the continuous body P into individual absorbent bodies P1 of which a longitudinal direction is a first direction. In the first transporting step S44, the individual absorbent bodies P1 after separation are transported such that the first direction and the transport direction of the absorbent bodies P1 coincide with each other. The second manufacturing step S5 includes a second separating step S51 and a second transporting step S53. In the second separating step S51, the continuous body P transported along the second transport path 23 is cut to separate the continuous body P into absorbent bodies P1. In the second transporting step S53, the individual absorbent bodies P1 after separation are transported such that the first direction and the transport direction of the absorbent bodies P1 are orthogonal to each other. By adopting such a configuration, the method for manufacturing an absorbent article enables absorbent articles of two different types to be manufactured from the continuous body P formed in a single step.
(3) A method for manufacturing an absorbent article according to a third aspect of the invention of the present application is the method for manufacturing an absorbent article according to the second aspect, in which the second manufacturing step S5 further includes an orientation changing step S52. In the orientation changing step S52, after separation in the second separating step S51, an orientation of each absorbent body P1, which is being transported so that the first direction and the transport direction of the absorbent bodies P1 coincide with each other, is rotated by 90° to make the first direction and the transport direction of the absorbent bodies P1 orthogonal to each other. By adopting such a configuration, in the second manufacturing step S5, the pants-type diaper 60 that differs from the tape-type diaper pants 50 manufactured in the first manufacturing step S4 can be manufactured from the continuous body P.
(4) A method for manufacturing an absorbent article according to a fourth aspect of the invention of the present application is the method for manufacturing an absorbent article according to any of the first to third aspects, in which the first manufacturing step S4 includes a side flap sheet manufacturing step S41 and a first combining step S42. In the side flap sheet manufacturing step S41, a first attachment member 81 to be attached around a person's waist is manufactured. In the first combining step S42, the first attachment member 81 and the absorbent body P1 are combined to manufacture a tape-type diaper as the first absorbent article 50. By adopting such a configuration, in the first manufacturing step S4, a tape-type diaper can be manufactured as the first absorbent article 50.
(5) A method for manufacturing an absorbent article according to a fifth aspect of the invention of the present application is the method for manufacturing an absorbent article according to any of the first to fourth aspects, in which the second manufacturing step S5 includes an attachment member sheet manufacturing step S54 and a second combining step S55. In the attachment member sheet manufacturing step S54, a second attachment member 83 to be attached around a person's waist is manufactured. In the second combining step S55, the second attachment member 83 and the absorbent body P1 are combined to manufacture a pants-type diaper as the second absorbent article 60. By adopting such a configuration, in the second manufacturing step S5, a pants-type diaper can be manufactured as the second absorbent article 60.
(6) A method for manufacturing an absorbent article according to a sixth aspect of the invention of the present application includes: a forming step S'1 of forming a continuous body P; a separating step S'2; a first manufacturing step S'4; a second manufacturing step S'5; and a switching step S'3. In the forming step S'1 of forming the continuous body P, the continuous body P having the plurality of absorbent bodies P1 in a state where the absorbent bodies P1 are connected in series is formed. In the separating step S'2, the continuous body P is cut and separated into individual absorbent bodies P1. In the first manufacturing step S'4, using the absorbent bodies P1, a tape-type diaper that is a first absorbent article 50 is manufactured. In the second manufacturing step S'5, using the absorbent bodies P1, a pants-type diaper that is a second absorbent article 60 that differs from the first absorbent article 50 is manufactured. In the switching step S'3, a supply destination of the absorbent bodies P1 separated in the separating step S'2 is switched between the first manufacturing step S4 and the second manufacturing step S5. By adopting such a configuration, the method for manufacturing an absorbent article enables reducing costs and floor space required for installing apparatuses.
(7) A method for manufacturing an absorbent article according to a seventh aspect of the invention of the present application is the method for manufacturing an absorbent article according to the sixth aspect, in which the first manufacturing step S'4 includes a first transporting step S'46. In the first transporting step S'46, the absorbent bodies P1 are transported such that the first direction being the longitudinal direction of the absorbent bodies P1 coincides with the transport direction of the absorbent bodies P1. The second manufacturing step S'5 includes a second transporting step S'52. In the second transporting step S'52, the absorbent bodies P1 are transported such that the first direction and the transport direction of the absorbent bodies P1 are orthogonal to each other. By adopting such a configuration, the method for manufacturing an absorbent article enables absorbent articles of two different types to be manufactured from the continuous body P formed by a single step.
(8) A method for manufacturing an absorbent article according to an eighth aspect of the invention of the present application is the method for manufacturing an absorbent article according to the seventh aspect, in which the second manufacturing step S'5 further includes an orientation changing step S'51. In the orientation changing step S'51, an orientation of the absorbent body P1, which is being transported so that the first direction and the transport direction of the absorbent bodies P1 coincide with each other, is rotated by 90° to make the first direction and the transport direction of the absorbent bodies P1 orthogonal to each other. By adopting such a configuration, in the second manufacturing step S'5, the pants-type diaper that differs from the tape-type diaper manufactured in the first manufacturing step S'4 can be manufactured.
(9) A method for manufacturing an absorbent article according to a ninth aspect of the invention of the present application is the method for manufacturing an absorbent article according to any of the sixth to eighth aspects, in which the first manufacturing step S'4 includes a side flap sheet manufacturing step S'47 and a first combining step S'48. In the side flap sheet manufacturing step S'47, the first attachment member 81 to be attached around a person's waist is manufactured. In the first combining step S'48, the first attachment member 81 and the absorbent body P1 are combined to manufacture a tape-type diaper as the first absorbent article 50. By adopting such a configuration, in the first manufacturing step S'4, a tape-type diaper can be manufactured as the first absorbent article 50.
(10) A method for manufacturing an absorbent article according to a tenth aspect of the invention of the present application is the method for manufacturing an absorbent article according to any of the sixth to ninth aspects, in which the second manufacturing step S'5 includes an attachment member sheet manufacturing step S'53 and a second combining step S'54. In the attachment member sheet manufacturing step S'53, a second attachment member 83 to be attached around a person's waist is manufactured. In the second combining step S'54, the second attachment member 83 and the absorbent body P1 are combined to manufacture a pants-type diaper as the second absorbent article 60. By adopting such a configuration, in the second manufacturing step S'5, a pants-type diaper can be manufactured as the second absorbent article 60.

### <Modifications>

Hereinafter, modifications of the embodiments described above will be described. Note that the following modifications may be combined as appropriate to the extent that they do not contradict each other.

### (1) Modification A

While two types of absorbent articles - the first absorbent article 50 and the second absorbent article 60 - are manufactured in the first manufacturing steps S4 and S'4 and the second manufacturing steps S5 and S'5, respectively, in the first and second embodiments described above, the number of types of absorbent articles to be manufactured is not particularly limited thereto. In the method for manufacturing an absorbent article according to the present invention, three or more types of absorbent articles may be manufactured.

### (2) Modification B

While different types of absorbent articles are manufactured in the first manufacturing steps S4 and S'4 and the second manufacturing steps S5 and S'5, respectively, in the first and second embodiments described above, the types of manufactured absorbent articles are not particularly limited thereto. In the method for manufacturing an absorbent article according to the invention of the present application, absorbent articles of the same type with partially different specifications such as size may be manufactured in the first manufacturing step S'4 and the second manufacturing step S'5.

### (3) Modification C

While the first manufacturing apparatus 30 that is a manufacturing apparatus of a tape-type diaper is arranged above the second manufacturing apparatus 40 that is a manufacturing apparatus of a pants-type diaper in the embodiments described above, the arrangement of the manufacturing apparatuses is not particularly limited thereto. For example, the first manufacturing apparatus 30 may be arranged below the second manufacturing apparatus 40. However, it is preferable to arrange a manufacturing apparatus with relatively lightweight components above a manufacturing apparatus with heavy components.

### (4) Modification D

While a tape-type diaper is manufactured in the first manufacturing steps S4 and S'4 and a pants-type diaper is manufactured in the second manufacturing steps S5 and S'5 in the embodiments described above, the articles to be manufactured are not particularly limited thereto. For example, a urine-absorbing pad may be manufactured in the first manufacturing steps S4 and S'4 and a tape-type diaper may be manufactured in the second manufacturing steps S5 and S'5.

When manufacturing a urine-absorbing pad in the first manufacturing steps S4 and S'4, the urine-absorbing pad as the first absorbent article 50 is formed by using the absorbent bodies P1 separated in the first separating step S43 or the separating step S'2 as the urine-absorbing pad.

### (5) Modification E

While a tape-type diaper is manufactured by the first manufacturing apparatus 30 and a pants-type diaper is manufactured by the second manufacturing apparatus 40 in the embodiments described above, the articles to be manufactured are not particularly limited thereto. For example, a sheet-type disposable sanitary napkin may be manufactured by the first manufacturing apparatus 30 and pants-type disposable menstrual underwear may be manufactured by the second manufacturing apparatus 40.

### (6) Modification F

While the absorbent bodies P1 are rotated in the orientation changing step S52 of the second manufacturing step S5 in the embodiments described above, operations with respect to the absorbent bodies P1 are not particularly limited thereto.

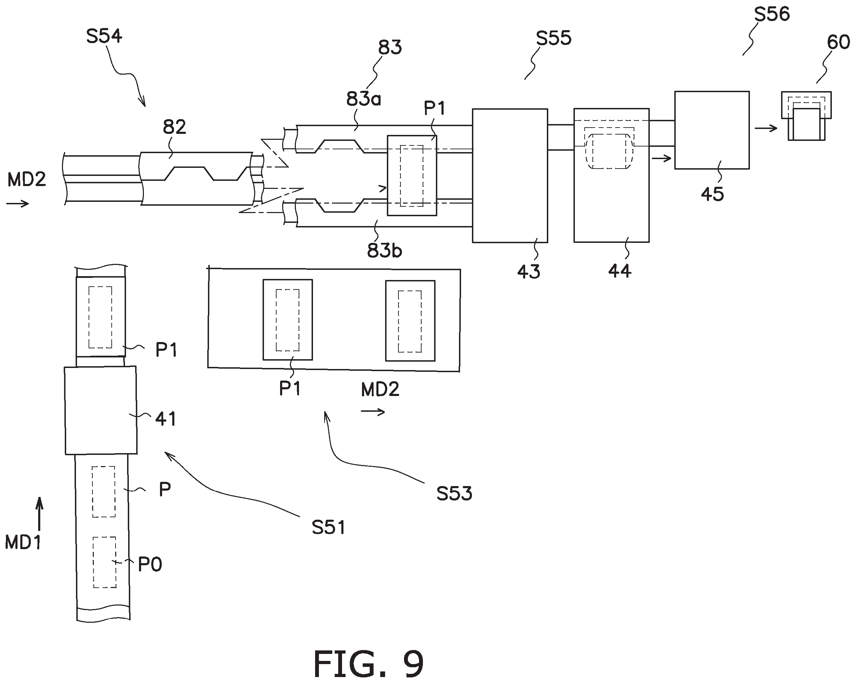

As shown in Fig. 9, the transport path itself of the absorbent bodies P1 after the second separating step S51 may be changed to a transport direction MD2. For example, the transport direction MD2 is a direction moved by 90° relative to a transport direction MD1 before the second separating step S51. In this case, before the second separating step S51, the first direction being the longitudinal direction of each absorbent body P1 and the transport direction MD1 of the absorbent body P1 are parallel to each other. After the second separating step S51, the first direction being the longitudinal direction of each absorbent body P1 and the transport direction MD2 of the absorbent body P1 are orthogonal to each other.

### <Appendix>

Finally, technical concepts grasped from the embodiments described above will be described.

A method for manufacturing an absorbent article according to the first aspect of the invention of the present application includes: a continuous body forming step; a setting step; a continuous body transporting step; a first manufacturing step; and a second manufacturing step. In the continuous body forming step, a continuous body having a plurality of absorbent bodies in a state where the absorbent bodies are connected in series is formed. In the setting step, a transport path of the continuous body is set to either a first transport path or a second transport path. In the continuous body transporting step, the continuous body is transported along the set transport path. In the first manufacturing step, the continuous body transported along the first transport path is cut and separated into individual absorbent bodies and a first absorbent article including the separated individual absorbent body is manufactured. In the second manufacturing step, the continuous body transported along the second transport path is cut and separated into individual absorbent bodies and, using the separated individual absorbent body, a second absorbent article of a different type from the first absorbent article is manufactured. By adopting such a configuration, the method for manufacturing an absorbent article enables reducing costs and floor space required for installing apparatuses.

A method for manufacturing an absorbent article according to a second aspect of the invention of the present application is the method for manufacturing an absorbent article according to the first aspect, in which the first manufacturing step includes a first separating step and a first transporting step. In the first separating step, the continuous body transported along the first transport path is cut to separate the continuous body into individual absorbent bodies of which a longitudinal direction is a first direction. In the first transporting step, the individual absorbent bodies after separation are transported such that the first direction and a transport direction of the absorbent bodies coincide with each other. The second manufacturing step includes a second separating step and a second transporting step. In the second separating step, the continuous body transported along the second transport path is cut to separate the continuous body into absorbent bodies. In the second transporting step, the individual absorbent bodies after separation are transported such that the first direction and the transport direction of the absorbent bodies are orthogonal to each other. By adopting such a configuration, the method for manufacturing an absorbent article enables absorbent articles of two different types to be manufactured from the continuous body formed by a single step.

A method for manufacturing an absorbent article according to a third aspect of the invention of the present application is the method for manufacturing an absorbent article according to the second aspect, in which the second manufacturing step further includes an orientation changing step. In the orientation changing step, after separation in the second separating step, an orientation of each absorbent body, which is being transported so that the first direction and the transport direction of the absorbent bodies coincide with each other, is rotated by 90° to make the first direction and the transport direction of the absorbent bodies orthogonal to each other. By adopting such a configuration, in the second manufacturing step, an absorbent article that differs from the absorbent article manufactured in the first manufacturing step can be manufactured from the continuous body.

A method for manufacturing an absorbent article according to a fourth aspect of the invention of the present application is the method for manufacturing an absorbent article according to any of the first to third aspects, in which the first manufacturing step includes a side flap sheet manufacturing step and a first combining step. In the side flap sheet manufacturing step, a first attachment member to be attached around a person's waist is manufactured. In the first combining step, the first attachment member and the absorbent body are combined to manufacture a tape-type diaper as the first absorbent article. By adopting such a configuration, in the first manufacturing step, a tape-type diaper can be manufactured as the first absorbent article.

A method for manufacturing an absorbent article according to a fifth aspect of the invention of the present application is the method for manufacturing an absorbent article according to any of the first to fourth aspects, in which the second manufacturing step includes an attachment member sheet manufacturing step and a second combining step. In the attachment member sheet manufacturing step, a second attachment member to be attached around a person's waist is manufactured. In the second combining step, the second attachment member and the absorbent body are combined to manufacture a pants-type diaper as the second absorbent article. By adopting such a configuration, in the second manufacturing step, a pants-type diaper can be manufactured as the second absorbent article.

A method for manufacturing an absorbent article according to a sixth aspect of the invention of the present application includes: a continuous body forming step; a separating step; a first manufacturing step; a second manufacturing step; and a switching step. In the continuous body forming step, a continuous body having a plurality of absorbent bodies in a state where the absorbent bodies are connected in series is formed. In the separating step, the continuous body is cut and separated into individual absorbent bodies. In the first manufacturing step, using the absorbent bodies, a first absorbent article is manufactured. In the second manufacturing step, using the absorbent bodies, a second absorbent article that differs from the first absorbent article is manufactured. In the switching step, a supply destination of the absorbent bodies separated in the separating step is switched between the first manufacturing step and the second manufacturing step. By adopting such a configuration, the method for manufacturing an absorbent article enables reducing costs and floor space required for installing apparatuses.

A method for manufacturing an absorbent article according to a seventh aspect of the invention of the present application is the method for manufacturing an absorbent article according to the sixth aspect, in which the first manufacturing step includes a first transporting step. In the first transporting step, the absorbent bodies are transported such that the first direction being the longitudinal direction of the absorbent bodies coincides with the transport direction of the absorbent bodies. The second manufacturing step includes a second transporting step. In the second transporting step, the absorbent bodies are transported such that the first direction and the transport direction of the absorbent bodies are orthogonal to each other. By adopting such a configuration, the method for manufacturing an absorbent article enables absorbent articles of two different types to be manufactured from the continuous body formed in a single step.

A method for manufacturing an absorbent article according to an eighth aspect of the invention of the present application is the method for manufacturing an absorbent article according to the seventh aspect, in which the second manufacturing step further includes an orientation changing step. In the orientation changing step, an orientation of the absorbent body, which is being transported so that the first direction and the transport direction of the absorbent bodies coincide with each other, is rotated by 90° to make the first direction and the transport direction of the absorbent bodies orthogonal to each other. By adopting such a configuration, in the second manufacturing step, an absorbent article that differs from the absorbent article manufactured in the first manufacturing step can be manufactured.

A method for manufacturing an absorbent article according to a ninth aspect of the invention of the present application is the method for manufacturing an absorbent article according to any of the sixth to eighth aspects, in which the first manufacturing step includes a side flap sheet manufacturing step and a first combining step. In the side flap sheet manufacturing step, a first attachment member to be attached around a person's waist is manufactured. In the first combining step, the first attachment member and the absorbent body are combined to manufacture a tape-type diaper as the first absorbent article. By adopting such a configuration, in the first manufacturing step, a tape-type diaper can be manufactured as the first absorbent article.

A method for manufacturing an absorbent article according to a tenth aspect of the invention of the present application is the method for manufacturing an absorbent article according to any of the sixth to ninth aspects, in which the second manufacturing step includes an attachment member sheet manufacturing step and a second combining step. In the attachment member sheet manufacturing step, a second attachment member to be attached around a person's waist is manufactured. In the second combining step, the second attachment member and the absorbent body are combined to manufacture a pants-type diaper as the second absorbent article. By adopting such a configuration, in the second manufacturing step, a pants-type diaper can be manufactured as the second absorbent article.

### Reference Signs List

- 100: System for manufacturing absorbent article
- 10: Absorbent body manufacturing apparatus
- 20: Switching apparatus
- 22: First transport path
- 23: Second transport path
- 30, 30': First manufacturing apparatus
- 40, 40': Second manufacturing apparatus
- 50: First absorbent article
- 60: Second absorbent article
- P: Continuous body of absorbent body
- P1: Absorbent body
- S1: Continuous body forming step
- S2: Setting step
- S3: Continuous body transporting step
- S4: First manufacturing step
- S5: Second manufacturing step
- S'1: Continuous body forming step
- S'2: Separating step
- S'3: Switching step
- S'4: First manufacturing step
- S'5: Second manufacturing step

## Claims

1. A method for manufacturing an absorbent article, the method comprising:
a continuous body forming step of forming a continuous body having a plurality of absorbent bodies in a state where the absorbent bodies are connected in series;
a setting step of setting a transport path of the continuous body to either a first transport path or a second transport path;
a continuous body transporting step of transporting the continuous body along the set transport path;
a first manufacturing step of cutting and separating the continuous body, transported along the first transport path, into the individual absorbent bodies and manufacturing a first absorbent article including the separated individual absorbent body; and
a second manufacturing step of cutting and separating the continuous body, transported along the second transport path, into the individual absorbent bodies and, using the separated individual absorbent body, manufacturing a second absorbent article of a different type from the first absorbent article.

2. The method for manufacturing an absorbent article according to claim 1, wherein the first manufacturing step includes:
a first separating step of cutting the continuous body transported along the first transport path to separate the continuous body into the individual absorbent bodies of which a longitudinal direction is a first direction; and
a first transporting step of transporting the individual absorbent bodies after separation such that the first direction and a transport direction of the absorbent bodies coincide with each other, and
the second manufacturing step includes:
a second separating step of cutting the continuous body transported along the second transport path to separate the continuous body into the absorbent bodies; and
a second transporting step of transporting the individual absorbent bodies after separation such that the first direction and the transport direction of the absorbent bodies are orthogonal to each other.

3. The method for manufacturing an absorbent article according to claim 1 or 2, wherein
the second manufacturing step further includes an orientation changing step of, after separation in the second separating step, rotating by 90° an orientation of each of the absorbent bodies being transported so that the first direction and the transport direction of the absorbent bodies coincide with each other to make the first direction and the transport direction of the absorbent bodies orthogonal to each other.

4. The method for manufacturing an absorbent article according to any one of claims 1 to 3, wherein
the first manufacturing step includes:
a side flap sheet manufacturing step of manufacturing a first attachment member to be attached around a person's waist; and
a first combining step of combining the first attachment member and the absorbent body to manufacture a tape-type diaper as the first absorbent article.

5. The method for manufacturing an absorbent article according to any one of claims 1 to 4, wherein
the second manufacturing step includes:
an attachment member sheet manufacturing step of manufacturing a second attachment member to be attached around a person's waist; and
a second combining step of combining the second attachment member and the absorbent body to manufacture a pants-type diaper as the second absorbent article.

6. A method for manufacturing an absorbent article, the method comprising:
a continuous body forming step of forming a continuous body of absorbent bodies in a state where a plurality of the absorbent bodies are connected in series;
a separating step of cutting the continuous body to separate the continuous body into the individual absorbent bodies;
a first manufacturing step of manufacturing a first absorbent article by using the absorbent bodies;
a second manufacturing step of manufacturing a second absorbent article that differs from the first absorbent article by using the absorbent bodies; and
a switching step of switching a supply destination of the absorbent bodies, separated in the separating step, between the first manufacturing step and the second manufacturing step.

7. The method for manufacturing an absorbent article according to claim 6, wherein
the first manufacturing step includes a first transporting step of transporting the absorbent bodies such that a first direction, which is a longitudinal direction of the absorbent bodies, coincides with a transport direction of the absorbent bodies; and
the second manufacturing step includes a second transporting step of transporting the absorbent bodies such that the first direction and the transport direction of the absorbent bodies are orthogonal to each other.

8. The method for manufacturing an absorbent article according to claim 6 or 7, wherein
the second manufacturing step further includes an orientation changing step of rotating by 90° an orientation of the absorbent bodies being transported so that the first direction and the transport direction of the absorbent bodies coincide with each other to make the first direction and the transport direction of the absorbent bodies orthogonal to each other.

9. The system for manufacturing an absorbent article according to any one of claims 6 to 8, wherein
the first manufacturing step includes:
a side flap sheet manufacturing step of manufacturing a first attachment member to be attached around a person's waist; and
a first combining step of combining the first attachment member and the absorbent body to manufacture a tape-type diaper as the first absorbent article.

10. The system for manufacturing an absorbent article according to any one of claims 6 to 9, wherein
the second manufacturing step includes:
an attachment member sheet manufacturing step of manufacturing a second attachment member to be attached around a person's waist; and
a second combining step of combining the second attachment member and the absorbent body to manufacture a pants-type diaper as the second absorbent article.
